# DEMANDE DE BREVET EUROPEEN

(11) **EP 2 078 495 A1**
(43) Date de publication de la demande: **15.07.2009**
(21) Numéro de dépôt: 09150526.3
(22) Date de dépôt: 14.01.2009
(51) Int. Cl.: A61B 8/08, A61B 8/06

(54) **Porte sonde échographique ultrasonore pour gestes echo guides**

(30) Priorité: 14.01.2008 FR 0800188; 07.03.2008 FR 0801254
(71) Demandeur: Grimaldi, Nathalie, 38320 Poisat (FR)
(72) Inventeur: Grimaldi, Nathalie, 38320 Poisat (FR)
(74) Mandataire: Verriest, Philippe

(57) **Abrégé**

Dispositif (1) pour tenir une sonde échographique ultrasonore (2) destinée à explorer une région du corps **caractérisé en ce qu**'il comporte un support principal (4), comportant une première partie (40) adaptée au membre (5) du patient et une seconde partie (41) prolongeant la première partie et formant un angle par rapport à celle-ci de façon à s'éloigner du membre du patient, un moyen de maintien (6) du support principal (4) au membre (5) du patient, au moins un support de sonde (3) sur lequel est fixée la sonde échographique ultrasonore (2), le support de sonde (3) étant fixé sur la seconde partie du support principal de façon à diriger la sonde vers la région du corps à explorer et à ce que la première partie du support et les moyens de maintien soient décalés par rapport de la région du corps à explorer.

## Description

La présente invention, concerne le domaine médical et plus particulièrement un dispositif porte sonde échographique ultrasonore qui permet de maintenir la sonde ultrasonore en position fonctionnelle en libérant les deux mains de l'opérateur afin de réaliser d'autres gestes écho guidés sur le corps humain ou autres.

La réalisation de gestes sous contrôle échographique apporte un gain de sûreté dans la prise en charge thérapeutique par rapport aux gestes réalisés sans ce contrôle. Ainsi l'écho sclérothérapie qui concerne l'injection d'un produit sclérosant dans une veine sous contrôle échographique apporte un gain de sécurité par rapport à la sclérothérapie simple.

Lors de la réalisation d'une sclérothérapie écho guidée il convient au préalable de réaliser une bonne exploration écho doppler, de déterminer le choix du site le plus pertinent pour faire l'injection, la ponction de la veine écho guidée avec la réalisation d'un reflux de sang dans la seringue, seule certitude d'être dans la veine, et l'injection du produit sclérosant totalement sous écho contrôle avec une vérification de la répartition du produit post injection et la position de l'aiguille.

En écho sclérothérapie, il existe des gestes incomplets tels que l'écho repérage simple puis l'injection sans écho contrôle, ou l'écho repérage et mise en place de l'aiguille écho guidée sans ponction suivi d'une injection avec ou sans écho contrôle. Ces gestes incomplets sont la conséquence d'une difficulté pour l'opérateur de manipuler la seringue d'une seule main. La sonde échographique ultrasonore est traditionnellement tenue d'une seule main, cependant s'il existe d'autres gestes à réaliser ceux-ci doivent être faits avec l'unique main libre rendant alors le geste difficile à effectuer. La technique de l'écho sclérothérapie présente une difficulté majeure qui est la manipulation de la seringue avec une seule main avec la réalisation entre d'autre d'un reflux de sang dans la seringue et l'injection du produit. Ce geste technique peut être réalisable pour certains opérateurs mais beaucoup moins aisé pour d'autres.

La présente invention permet de remédier à cet inconvénient.

A cet effet, la présente invention a pour objet, un dispositif pour tenir une sonde échographique ultrasonore destinée à explorer une région du corps **caractérisé en ce qu**'il comporte un support principal, comportant une première partie adaptée au membre du patient et une seconde partie prolongeant la première partie et formant un angle par rapport à celle-ci de façon à s'éloigner du membre du patient, un moyen de maintien du support principal au membre du patient, au moins un support de sonde sur lequel est fixée la sonde échographique ultrasonore, le support de sonde étant fixé sur la seconde partie du support principal de façon à diriger la sonde vers la région du corps à explorer et à ce que la première partie du support et les moyens de maintien soient décalés par rapport de la région du corps à explorer.

Ce dispositif peut être utilisé par exemple pour des gestes d'écho sclérothérapie. Ainsi, l'opérateur peut amener la pointe de l'aiguille dans la varice à scléroser avec ses deux mains, la maintenir dans cette position durant toute la durée du geste en effectuant un reflux veineux dans la seringue, seule certitude d'être en intra veineux, puis injecter le produit sclérosant dans la veine. Le dispositif permet de faire tout le geste d'écho sclérothérapie sous écho contrôle.

Cette disposition permet donc de maintenir le dispositif et la sonde en position fonctionnelle en contact avec la peau en regard de la région à explorer pouvant alors libérer les deux mains de l'opérateur afin qu'il puisse réaliser des gestes thérapeutiques écho guidés avec plus d'aisance et de sûreté. Les gestes thérapeutiques peuvent notamment consister en des ponctions, des prélèvements, des injections. Le dispositif est solidaire de la partie du corps à traiter et donc mobile avec elle, permettant si la personne bouge de rester fixe par rapport au membre traité. Le dispositif peut être destiné à être appliqué sur différentes régions du corps humain et notamment les membres inférieurs. Le support de sonde est porté par un support principal destiné à être fixé au membre d'un patient grâce à des moyens de maintien ce qui permet de découpler l'effort de pression de fixation du dispositif au corps et l'effort de pression appliqué sur la sonde échographique ultrasonore, qui est réglable.

Selon un mode de réalisation, un organe complémentaire est relié à la première partie du support principal de façon à ménager entre l'organe complémentaire et le support principal un passage pour le moyen de maintien et des moyens de réglage destinés à assurer un coincement ou un coulissement des moyens de maintien par rapport au support principal.

Cette disposition permet de faire coulisser les moyens de maintien au travers de ce passage afin d'ajuster la position du dispositif sur le membre du patient. Cet organe complémentaire peut s'étendre sensiblement parallèlement et à une certaine distance de la première partie du support principal de façon à ménager le passage pour le moyen de maintien. Il peut par exemple être constitué par une première partie de plaque prolongée par une seconde partie de plaque.

Selon un mode de réalisation, la première partie a une forme cylindrique et/ou concave.

Selon un mode de réalisation, le moyen de maintien est une sangle.

Cette disposition permet de découpler l'effort de pression de fixation du dispositif au corps et l'effort de pression appliqué sur la sonde échographique ultrasonore dont l'orientation est réglable.

Selon ce même mode de réalisation, la sangle comporte une boucle de connexion et de déconnexion.

Cette boucle de connexion et de déconnexion est comprise dans le moyen de maintien qui entoure en aval la région du corps à explorer et la laisse accessible pour y effectuer des gestes thérapeutiques sous contrôle visuel.

Avantageusement, le support de sonde comporte une plaque dont la face en regard de la seconde partie du support principal comprend des moyens de retenue destinés à retenir le corps de la sonde échographique ultrasonore. Ces moyens de retenu du corps de la sonde échographique peuvent être constitués par deux sangles.

Selon un mode de réalisation, la face en regard de la seconde partie du support principal est revêtue sur la face destinée à recevoir la sonde échographique ultrasonore d'une couche de mousse ou similaire.

Selon un mode de réalisation, la plaque du support de sonde est fixée sur la paroi de la seconde partie du support principal grâce à du textile agrippant.

Cette disposition permet de positionner la plaque du support à hauteur voulue. De cette façon l'effort de fixation du support principal sur le membre est découplé de l'effort de pression appliqué sur la sonde, ce permet de ne pas comprimer la partie du corps du patient examinée dont la veine.

Selon un mode de réalisation, l'organe complémentaire comprend un organe de retenue comprenant un passage destiné à retenir le câble de la sonde d'échographie ultrasonore.

Cet organe de retenue comprend un passage destiné à retenir le câble de la sonde d'échographie ultrasonore ce qui permet de reporter le poids du câble sur le support principal, ce qui supprime les efforts parasites sur la sonde échographique ultrasonore permettant ainsi son positionnement sans effort de pression parasite sur la partie du corps à examiner.

Selon un mode de réalisation, l'organe complémentaire est relié à la première partie du support principal sur une extrémité de sa première partie à l'aide d'un système de fixation, tandis que sa seconde partie est retenue sur la seconde partie du support principal par un moyen de réglage réalisée dans la seconde partie de l'organe complémentaire coopérant avec un système de verrouillage.

Le système de fixation peut être constitué par exemple par une vis, le moyen de réglage par une rainure verticale réalisée dans la seconde partie de l'organe complémentaire et le système de verrouillage par une came.

Ainsi, grâce au déplacement vertical de la seconde partie de l'organe complémentaire, le praticien pourra assurer le serrage de la sangle dans la zone du passage une fois la position du support définie.

Selon un mode de réalisation, le support principal se présente sous la forme d'une plaque en forme de « L » formant une équerre de retenue pour le support de sonde.

Ainsi, le support principal peut être constitué par une partie de plaque horizontale de forme cylindrique ou autre adaptée au membre du patient, prolongée verticalement par une partie de plaque verticale, formant ainsi une équerre de retenue pour le support de sonde, tandis qu'il comprend des moyens de maintien, destinés à fixer le support sur le membre du patient, de façon à être solidaire du corps du patient et de ses mouvements ce qui permet à la sonde d'échographie ultrasonore de rester fixe par rapport à la partie du corps à examiner.

L'organe complémentaire dans ce cas peut lui aussi se présenter sous la forme d'une plaque en forme de « L » avec une partie de plaque horizontale prolongée verticalement par une partie de plaque verticale formant ainsi une équerre, tandis que la partie horizontale de l'organe complémentaire est disposée et fixée à la partie horizontale du support principal pour s'étendre parallèlement à une certaine distance de façon à ménager un passage pour la sangle.

Selon un mode de réalisation, le support principal comporte une articulation entre la première partie et la seconde partie.

Cette articulation permet d'orienter la sonde selon une direction supplémentaire donné par un angle d'ouverture entre la première et la seconde partie du support principal. Dans ce mode de réalisation, cette articulation peut comporter de façon connue un moyen de blocage en position comme par exemple un axe amovible dentelé.

Dans ce cas, l'organe complémentaire peut lui aussi se présenter sous la forme d'une plaque articulée entre la première partie de plaque et la seconde partie de plaque. La plaque de l'organe complémentaire étant fixé au support principal, celui ne nécessite pas obligatoirement de moyen de blocage en position venant en supplément de celui du support principal.

Selon un mode de réalisation, la seconde partie du support principal et/ou le support de sonde comporte une plaque supplémentaire avec du textile agrippant sur une de ses faces maintenue par un système articulé blocable en position par un moyen de serrage.

Cette disposition permet d'ajuster l'orientation de la sonde ultrasonore qui peut rester mobile, au cours de l'examen en fonction du besoin, avec tous les plans de coupe choisis qui dépendent de la position de la sonde sur la peau par rapport à l'axe des organes à explorer.

Le système articulé de cette plaque peut consister de manière connue en une charnière avec un prolongement transversal dentelé de son axe amovible venant accroché le corps de la charnière et pouvant être maintenu en position par exemple par un ressort ou par un écrou à oreilles facilitant son serrage.

L'invention constitue ainsi un dispositif simple, réglable, aisément manipulable et de mise en place facile. Le dispositif selon l'invention est particulièrement destiné à réaliser de l'écho sclérothérapie mais non exclusivement.

D'autres caractéristiques et avantages de l'invention apparaîtront au cours de la description suivante d'une des formes de réalisation, donnée à titre d'exemple non limitatif, en regard des dessins annexés.

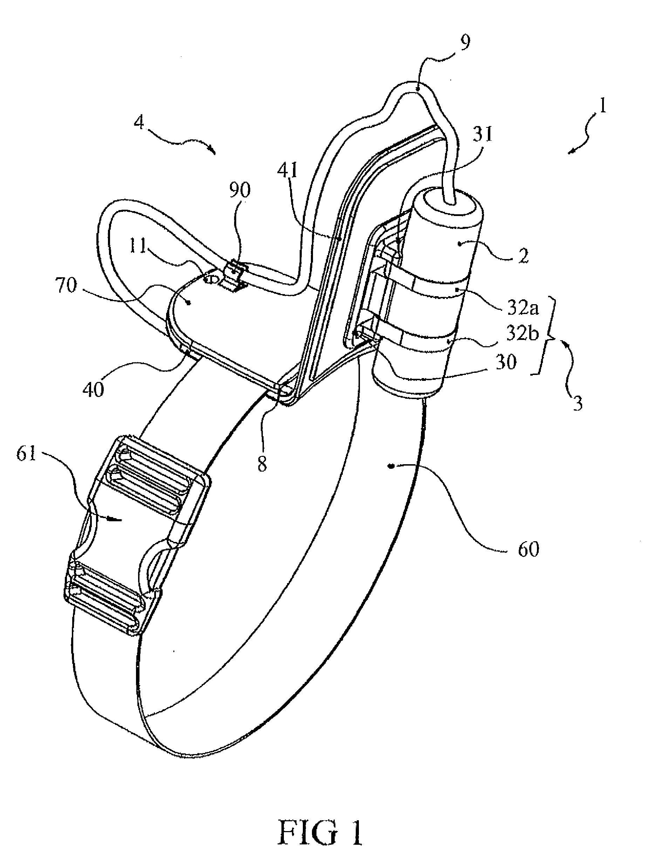

La figure 1 est une vue en perspective avant du porte sonde avec la sonde d'échographie ultrasonore.

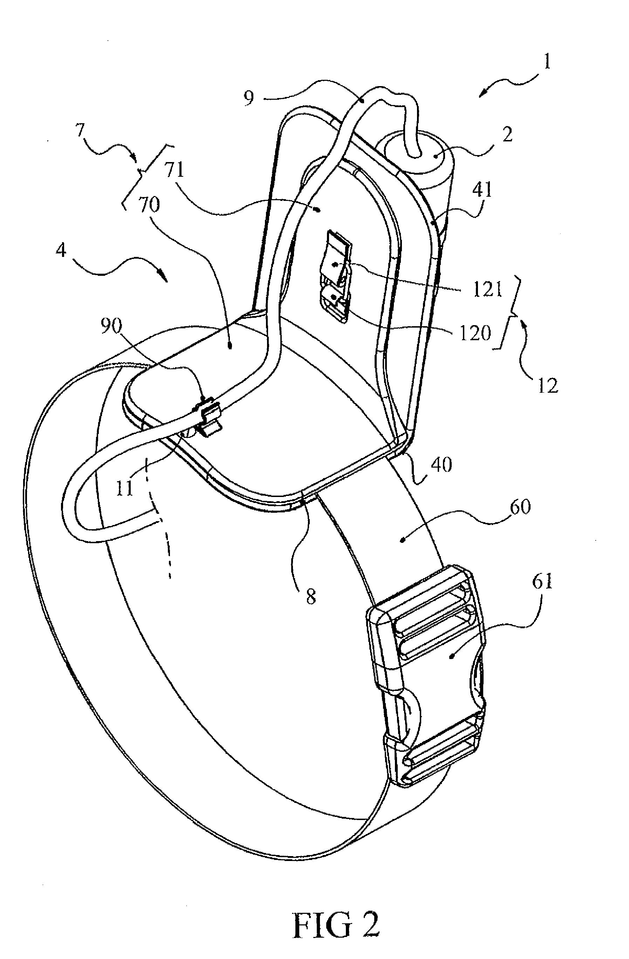

La figure 2 est une vue en perspective arrière du porte sonde avec la sonde d'échographie ultrasonore.

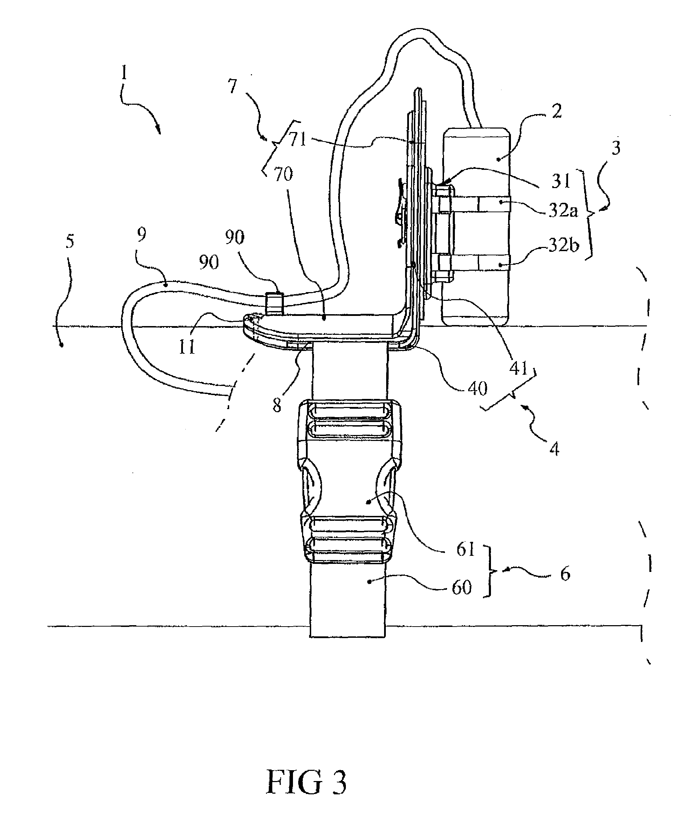

La figure 3 est une vue latérale montrant le porte sonde installé sur le membre du patient.

Les figures 4a, 4b, 4b', 5a et 5b sont des vues illustrant le mode opératoire de mise en place du dispositif.

Les figures 6a , 6b et 7 illustrent des modes de réalisation du dispositif avec des moyens d'articulation.

Le porte sonde de l'invention portant la référence générale (1) est donc destiné à maintenir une sonde échographique ultrasonore (2), et comprend au moins un support de sonde (3) porté par un support principal (4) destiné à être fixé au membre (5) d'un patient grâce à un moyen de maintien (6).

Le support principal (4) se présente sous la forme d'une plaque en forme de « L », constituée par une partie de plaque horizontale (40) prolongée verticalement par une partie de plaque verticale (41) formant ainsi une équerre de retenue pour le support de sonde (3).

Comme nous l'avons écrit précédemment, le support principal (4) est fixé au membre (5) du patient, par un moyen de maintien (6) constitué par exemple par une sangle (60) avantageusement large comprenant avantageusement une boucle de connexion et de déconnexion (61). Cette sangle (60) est par exemple réalisée en matériau textile. Cette sangle (60) est destinée à entourer le membre du patient dans une zone située en aval de la zone à explorer.

Au support principal (4) est associé un organe complémentaire (7) qui se présente sous la forme d'une plaque en forme de « L », constituée par une partie de plaque horizontale (70) prolongée verticalement par une partie de plaque verticale (71) formant ainsi une équerre. Notons que la partie horizontale (70) de l'organe complémentaire (7) est disposé et fixé à la partie horizontale (40) du support principal (4) à l'aide d'un système de fixation par une vis ou autre (11) pour s'étendre parallèlement à une certaine distance de façon à ménager un passage (8) pour la sangle (60). Tandis que la partie verticale (71) de l'organe complémentaire (7) est retenue sur la partie verticale (41) du support principal par un moyen de réglage (12) constitué par exemple par une rainure verticale (120) réalisée dans la partie verticale (71) de l'organe complémentaire (7) coopérant avec un système de verrouillage, comme par exemple une came (121) ou tout autre dispositif approprié. Ainsi grâce au déplacement vertical de la partie verticale de l'organe complémentaire (7) le praticien pourra assurer le serrage de la sangle dans la zone de passage (8) une fois que la position de la sonde échographique ultrasonore sur le membre est effectuée (5).

Notons aussi que la partie horizontale (70) de l'organe complémentaire (7) comprend un organe de retenue (90) comprenant un passage destiné à retenir le câble (9) de la sonde échographique ultrasonore (2).

Ajoutons que la partie horizontale (40) du support principal (4) ainsi que la partie horizontale (70) de l'organe complémentaire (7), sont constituées par une portion de paroi cylindrique ou autre afin d'avoir une forme complémentaire à celle du membre du patient. La face de la partie de plaque verticale (41) du support principal en regard du support de sonde (30) peut avoir une forme plane, concave, convexe ou une combinaison de ces formes.

Le support de sonde (3) est constitué par une plaque (30) revêtue avantageusement sur la face destinée à recevoir la sonde échographique ultrasonore d'une couche de mousse (31) ou similaire permettant une bonne retenue du corps de la sonde échographique ultrasonore. Cette même face comprend par ailleurs des moyens de retenue destinés à retenir le corps de la sonde échographique ultrasonore. Ces moyens sont par exemple constitués par deux sangles (32a, 32b) dont les extrémités comprennent par exemple du textile agrippant. Bien entendu la retenue de la sonde, pourrait être assurée par d'autres moyens. La face de la plaque (30) du support de sonde en regard de la partie de plaque verticale (41) du support principal peut avoir une forme plane, concave, convexe ou une combinaison de ces formes. De cette façon la face de la plaque (30) du support de sonde et la partie de plaque verticale (41) du support principal ont une forme adaptée pour permettre l'inclinaison de la sonde échographique ultrasonore (2).

Par ailleurs, la plaque (30) du support de sonde (3) est fixée sur la paroi de la partie verticale (41) du support principal (4) grâce à du textile agrippant (13), ce qui permet de positionner la plaque du support de sonde à la hauteur voulue et de pouvoir maintenir la sonde échographique ultrasonore avec un angle choisi par rapport à la partie du corps à examiner.

Les plaques constituant le support de sonde et le support principal, sont par exemple réalisées en matériau composite, mais il pourrait en être bien entendu autrement comme par exemple en matière plastique, ou autre.

A titre d'exemple bien entendu non limitatif :
- La plaque (30) du support de sonde (3) pourrait avoir une hauteur de 7 centimètres, une largeur de 5 centimètres et une épaisseur de 3 millimètres.
- La plaque (30) aura par exemple 4 trous permettant le passage de 2 sangles de 1 centimètre de hauteur et 20 centimètres de longueur avec un tissu adhésif adaptable en fonction de la taille de la sonde échographique ultrasonore.
- La partie horizontale (40) du support principal (4) pourrait par exemple avoir une longueur de 10 centimètres et une largeur de 7 centimètres tandis que son épaisseur serait par exemple de 3 millimètres.
- La partie verticale (41) du support principal (4) pourrait par exemple avoir une hauteur de 9 centimètres et une largeur de 6 centimètres tandis que son épaisseur serait par exemple de 3 millimètres.
- La partie horizontale (70) de l'organe complémentaire (7) pourrait par exemple avoir une longueur de 10 centimètres et une largeur de 7 centimètres, tandis que son épaisseur serait par exemple de 3 millimètres.
- La partie verticale (71) de l'organe complémentaire (7) pourrait par exemple avoir une hauteur de 8 centimètres et une largeur de 6 centimètres, tandis que son épaisseur serait par exemple de 3 millimètres.
- La sangle (60) réalisée en matériau textile voir élastique, pourrait avoir par exemple une largeur de 4 centimètres.

Le dispositif qui vient d'être décrit fonctionne comme suit :

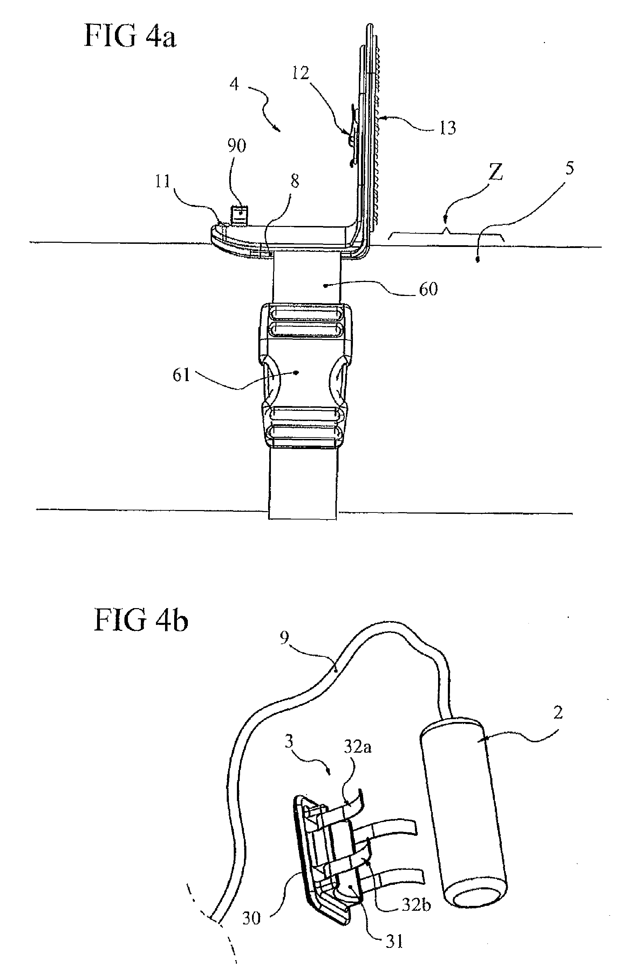

Le praticien fixe provisoirement le support principal (4) sur le membre du patient (5), grâce à la sangle de maintien (60), et ce bien entendu à un endroit du membre laissant libre la zone (Z) à explorer (figure 4a).

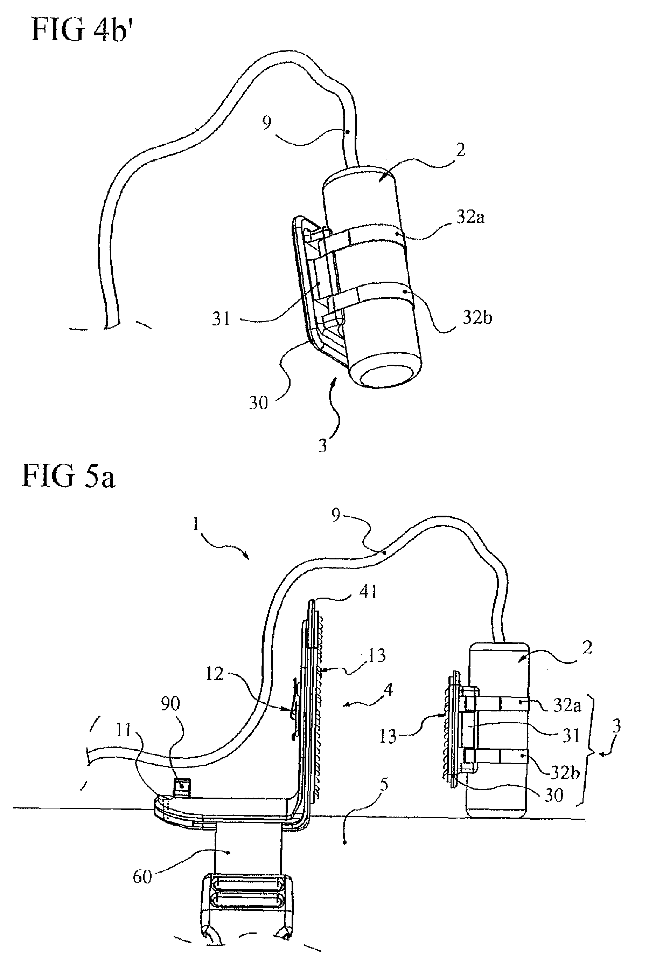

Le praticien fixe la sonde échographique ultrasonore (2) sur le support de sonde (3) grâce aux sangles de retenue (32a, 32b) (figure 4b, 4b').

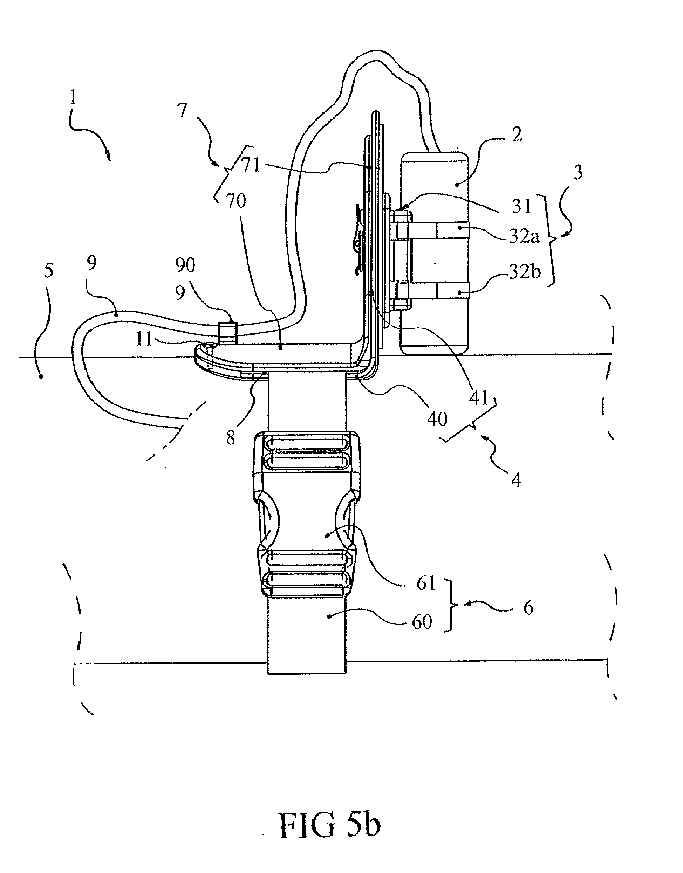

Le praticien applique alors la sonde échographique ultrasonore (2) sur la partie du corps du patient avec une application préalable d'un gel ou autre produit de couplage pour favoriser la transmission des ondes échographiques ultrasonores. Il définit le site le plus pertinent pour faire le geste d'écho sclérothérapie (figure 5).

Le praticien fixe sur le support principal (4) le support de sonde (3) avec la sonde échographique ultrasonore (2) grâce au tissu agrippant (13) et laisse en place la sonde échographique ultrasonore sans la tenir en libérant ses mains. Puis il fixe le câble (9) de la sonde échographique ultrasonore sur l'organe de retenue (90). L'opérateur peut bouger la sonde échographique ultrasonore pour l'orienter avec précision dans le plan choisi en fonction des besoins de l'examen. Le praticien une fois la zone définie pour le geste thérapeutique, peut fixer correctement le support principal (4) au membre du patient en provoquant le serrage de la sangle de maintien (60), en réduisant l'espace (8) par le déplacement vers le bas de l'organe complémentaire et le verrouillage de ce dernier par rapport au support principal (4), grâce à la came (121).

Puis il peut effectuer à l'aide de ses deux mains la ponction de la veine écho guidée avec la réalisation d'un reflux de sang dans la seringue et l'injection du produit sclérosant, ceci totalement sous écho contrôle. Puis la sonde échographique ultrasonore et son câble peuvent être libérés rapidement en séparant le support de sonde (3) du support principal (4) pour permettre une vérification de la répartition du produit post injection. Le support principal peut être alors retiré du membre du patient.

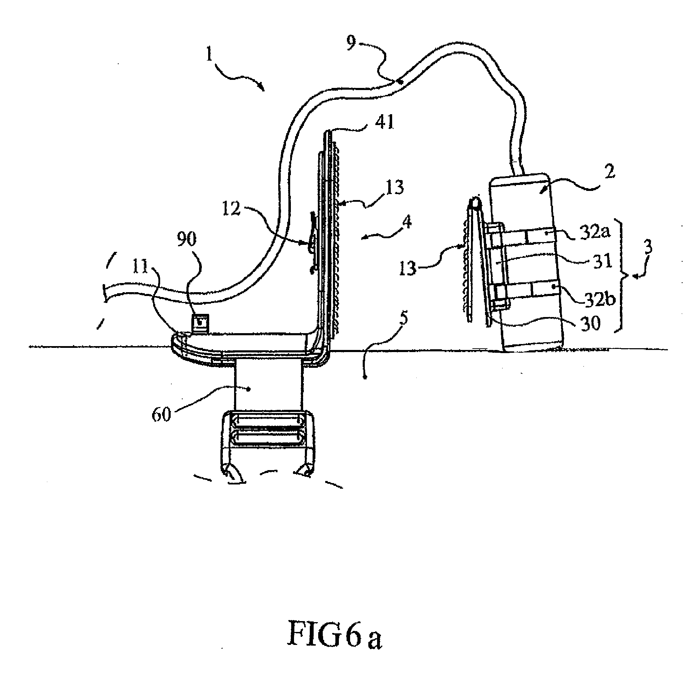

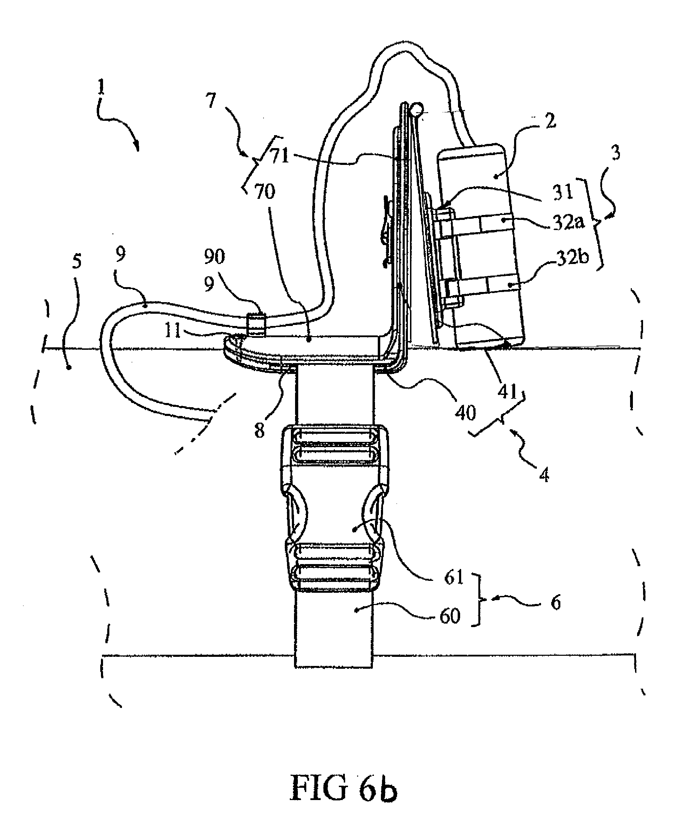

Selon une autre variante illustrée aux figures 6a et 6b, une plaque supplémentaire qui comporte sur une face du textile agrippant ou autre peut être intégrée à la partie verticale du support principal (41) et/ou au support de sonde 30 par un système articulé ou autre lui permettant d'être mobile et d'être bloquée en position grâce à un moyen de serrage approprié permettant ainsi au support de sonde 3 de pivoter pour positionner la sonde échographique ultrasonore suivant un angle choisi par rapport à la partie du corps à examiner.

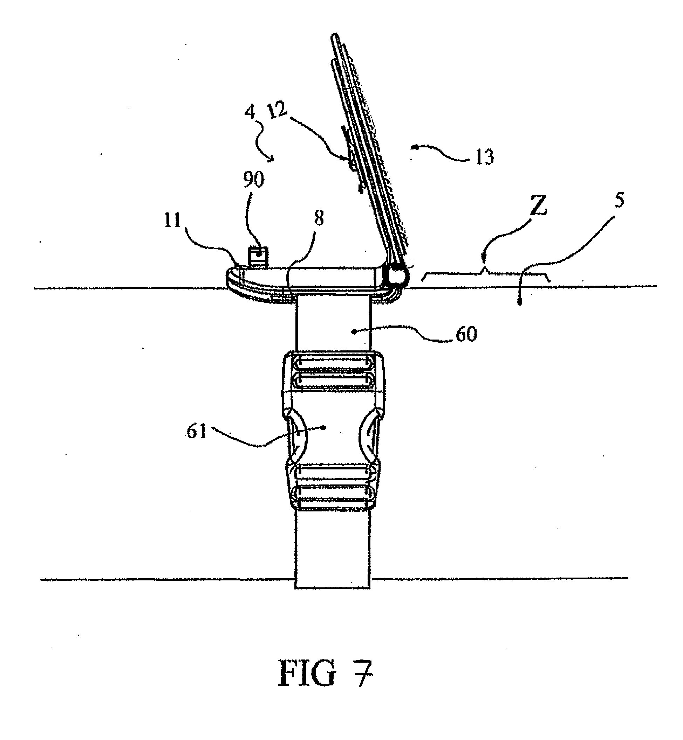

Selon une autre variante illustrée à la figure 7, le support principal 4 et l'organe complémentaire 7 se présentent sous la forme de plaques en forme de « L », constituées par les parties de plaques horizontales 40, 70 et les parties de plaques 41, 71 qui peuvent être articulées et bloquées en position grâce à un moyen de serrage approprié formant ainsi une fausse équerre de retenue à branches mobiles pour le support de sonde 3, pour permettre d'orienter la sonde échographique ultrasonore selon un angle choisi par rapport à la partie du corps à examiner.

Bien entendu, l'invention n'est pas limitée aux modes de réalisation qui viennent d'être décrits, qui n'ont été donnés qu'à titre d'exemple. Les premières parties 40, 70 du support principal 4 et de l'organe complémentaire 7 peuvent par exemple comprendre une portion formant un angle prédéterminé, à partir de laquelle s'étendent les secondes parties 41, 71 du support principal 4 et de l'organe complémentaire 7, lesdites premières parties 40, 70 étant reliées aux dites secondes parties 41, 71 par des moyens d'articulation.

## Revendications

1. Dispositif (1) pour tenir une sonde échographique ultrasonore (2) destinée à explorer une région du corps **caractérisé en ce qu'**il comporte :
- un support principal (4), comportant une première partie (40) adaptée au membre (5) du patient et une seconde partie (41) prolongeant la première partie et formant un angle par rapport à celle-ci de façon à s'éloigner du membre du patient,
- un moyen de maintien (6) du support principal (4) au membre (5) du patient,
- au moins un support de sonde (3) sur lequel est fixée la sonde échographique ultrasonore (2),
le support de sonde (3) étant fixé sur la seconde partie du support principal de façon à diriger la sonde vers la région du corps à explorer et à ce que la première partie du support et les moyens de maintien soient décalés par rapport à la région du corps à explorer.

2. Dispositif selon la revendication 1, dans lequel un organe complémentaire (7) est relié à la première partie (40) du support principal (4) de façon à ménager entre l'organe complémentaire et le support principal un passage (8) pour le moyen de maintien (6) et des moyens de réglage (12) destinés à assurer un coincement ou un coulissement des moyens de maintien par rapport au support principal.

3. Dispositif (1) selon l'une des revendications précédentes, dans lequel la première partie (40) a une forme cylindrique et/ou concave.

4. Dispositif (1) selon l'une des revendications précédentes, dans lequel le moyen de maintien (6) comprend une sangle (60).

5. Dispositif (1) selon la revendication 4, dans lequel la sangle (60) comporte une boucle de connexion et de déconnexion (61).

6. Dispositif (1) selon l'une des revendications précédentes, dans lequel le support de sonde (3) comporte une plaque (30) dont la face en regard de la seconde partie (41) du support principal (4) comprend des moyens de retenue (32a, 32b) destinés à retenir le corps de la sonde échographique ultrasonore (2).

7. Dispositif (1) selon la revendication 6, dans lequel la face en regard de la seconde partie (41) du support principal (4) est revêtue sur la face destinée à recevoir la sonde échographique ultrasonore (2) d'une couche de mousse (31) ou similaire.

8. Dispositif (1) selon l'une des revendications 6 à 7, dans lequel la plaque (30) du support de sonde (3) est fixée sur la paroi de la seconde partie (41) du support principal (4) grâce à du textile agrippant (13).

9. Dispositif (1) selon l'une des 2 à 8, dans lequel l'organe complémentaire (7) comprend un organe de retenue (90) comprenant un passage destiné à retenir le câble (9) de la sonde d'échographie ultrasonore (2).

10. Dispositif (1) selon l'une des revendications 2 à 8, dans lequel l'organe complémentaire (7) comporte une première partie de plaque (70) prolongée par une seconde partie de plaque (71), l'organe complémentaire (7) étant relié à la première partie (40) du support principal (4) sur une extrémité de sa première partie (70) à l'aide d'un système de fixation (11), tandis que sa seconde partie (71) est retenue sur la seconde partie (41) du support principal (4) par un moyen de réglage (12) réalisée dans la seconde partie (71) de l'organe complémentaire (7) coopérant avec un système de verrouillage (121).

11. Dispositif (1) selon l'une des revendications précédentes, dans lequel le support principal (4) se présente sous la forme d'une plaque en forme de « L » formant une équerre de retenue pour le support de sonde (3).

12. Dispositif (1) selon l'une des revendications 1 à 10, dans lequel le support principal (4) comprend une articulation entre la première partie (40) et la seconde partie (41).

13. Dispositif (1) selon l'une des revendications précédentes, dans lequel la seconde partie (41) du support principal (4) et/ou le support de sonde (3) comporte une plaque supplémentaire avec du textile agrippant sur une de ses faces maintenue par un système articulé blocable en position par un moyen de serrage.
